# DEMANDE DE BREVET EUROPEEN

(11) **EP 4 566 445 A1**
(43) Date de publication de la demande: **11.06.2025**
(21) Numéro de dépôt: 23215078.9
(22) Date de dépôt: 07.12.2023
(51) Int. Cl.: A01K 11/00

(54) **DISPOSITIF SCIENTIFIQUE D'ÉTUDE D'ANIMAUX, NOTAMMENT D'ANIMAUX MARINS**

(71) Demandeur: Blancpain SA, 1348 Le Brassus (CH); Andromède Océanologie Sàrl, 34130 Mauguio (FR)
(72) Inventeur: WINKLER, Yves, 3185 Schmitten (CH); HALLER, Timothée, 2019 Rochefort (CH); VONLANTHEN, Yannick, 2300 La Chaux-de-Fonds (CH); GUILBERT, Antonin, 34000 Montpellier (FR); BALLESTA, Laurent, 34130 Mauguio (FR)
(74) Mandataire: ICB SA

(57) **Abrégé**

Un aspect de l'invention concerne un dispositif d'étude d'animaux (100), projetable sur un animal pour un balisage et un recueil d'échantillon, comportant une flèche (1) qui comporte, coaxiaux et démontables, un corps de flèche (2), un dard creux tranchant (3) et un insert (5), le dard creux tranchant (3) comportant en partie arrière (22) des moyens d'appui pour un dispositif de propulsion et portant en partie avant (21) ce dard creux tranchant (3) apte à pénétrer la peau d'un animal lors d'un impact et carotter dans un alésage (4) un échantillon dudit animal, ce dard creux tranchant (3) portant extérieurement, en appui sur un premier épaulement frontal (6) du corps de flèche (2), l' insert (5) à fixer sous la peau dudit animal, le corps de flèche (2) comportant au niveau de la partie arrière (22) un deuxième épaulement (7) pour stopper la pénétration du corps de flèche (2) dans ledit animal.

## Description

### Domaine technique de l'invention

L'invention concerne un dispositif scientifique d'étude d'animaux, agencé pour être lancé par un dispositif de propulsion sur un animal pour un balisage et un recueil d'échantillon dudit animal, comportant une flèche.

L'invention concerne le domaine de l'expérimentation scientifique sur animaux vivants, et plus particulièrement sur animaux marins.

### Arrière-plan technologique

La pose d'une balise de suivi sur un animal vivant est une opération délicate et difficile. Les scientifiques souhaitent éviter de devoir endormir un animal, ou de devoir pêcher un animal marin ou aquatique, pour effectuer la pose d'une balise. Ainsi on éviterait de soumettre l'animal à un stress important.

Il en est de même pour le prélèvement d'échantillons de tissus, qui se révèle particulièrement difficile sur des animaux sauvages, en particulier parmi des espèces très sensibles, comme les requins par exemple. Ce prélèvement a pour objectif, par exemple, de connaître l'état sanitaire de l'animal, mais aussi les éventuelles pathologies ou anomalies génétiques.

Des essais ont été menés avec succès pour effectuer le balisage ou le prélèvement directement dans le milieu où vit l'animal, par exemple le milieu marin, et à distance raisonnable de l'animal, à l'aide d'un dispositif de propulsion particulier, pour réduire significativement le stress induit par l'opération. D'autres essais ont été réalisés pour jumeler les opérations de pose de balise et de prélèvement d'échantillon, de façon à limiter l'investigation à un contact unique avec l'animal toujours dans le but de réduire l'impact sur son bien-être. En particulier on connaît une flèche permettant de réaliser en même temps la pose d'une balise et un prélèvement biologique, sur de telles espèces. Toutefois, la méthode n'est pas infaillible, car on se heurte souvent à une absence de pénétration dans la peau de l'animal, ce qui ne permet ni balisage ni recueil d'échantillon, ou encore à une mauvaise accroche de la chair de l'animal dans le tube de prélèvement mais toutefois avec un balisage correct.

Il s'agit donc de fiabiliser un dispositif expérimental pour réussir à coup sûr la pose d'une balise et un recueil fiable d'échantillon en une seule opération.

### Résumé de l'invention

L'invention se propose d'améliorer le taux de réussite lors des tirs mixtes de balisage/prélèvement sur des animaux, plus particulièrement des animaux marins, plus particulièrement encore des requins, comme par exemple des requins marteaux.

A cet effet, l'invention concerne un dispositif scientifique d'étude d'animaux, agencé pour être lancé par un dispositif de propulsion sur un animal pour un balisage et un recueil d'échantillon dudit animal, comportant une flèche.

Selon l'invention, ladite flèche comporte, sensiblement coaxiaux autour d'une direction longitudinale et démontables l'un par rapport à l'autre, un corps de flèche, un dard creux tranchant et au moins un insert, ledit corps de flèche comportant dans une partie arrière des moyens d'appui pour un dit dispositif de propulsion et portant en appui longitudinal dans une partie avant ledit dard creux tranchant formant un tube de prélèvement agencé pour pénétrer la peau d'un animal lors d'un impact et carotter dans un alésage dudit dard creux tranchant un échantillon dudit animal, ledit dard creux tranchant portant extérieurement ledit au moins un insert agencé pour être fixé sous la peau dudit animal, ledit au moins un insert étant amovible par rapport audit corps de flèche et audit dard creux tranchant et agencé pour reposer en appui sur un premier épaulement frontal dudit corps de flèche, lequel corps de flèche comporte au niveau de ladite partie arrière un deuxième épaulement agencé pour stopper la pénétration dudit corps de flèche dans ledit animal.

### Brève description des figures

Les buts, avantages et caractéristiques de l'invention apparaîtront mieux à la lecture de la description détaillée qui va suivre, en référence aux dessins annexés, où :

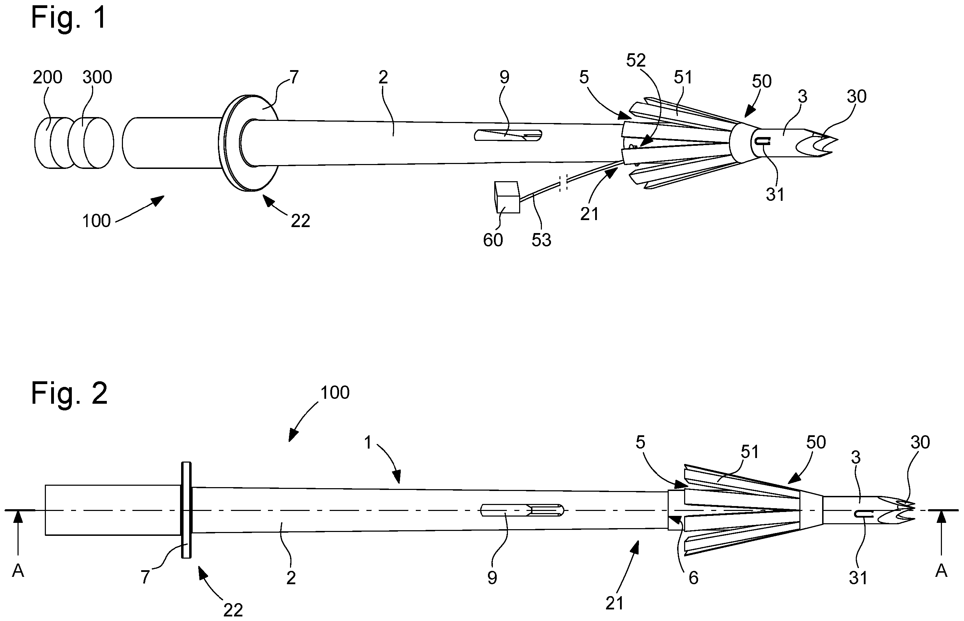

- la figure 1 illustre, de façon schématisée et en perspective, un dispositif scientifique d'étude d'animaux comportant, schématisés de gauche à droite de la figure, des moyens de propulsion agencés pour projeter une flèche sur une zone d'observation ou/et de balisage d'un animal, des moyens de retrait pour la récupération de cette flèche après le prélèvement d'un échantillon, et une flèche propre à l'invention, en plusieurs parties assemblées, et comportant, de l'arrière vers l'avant, un corps de flèche et un dard creux tranchant, formant réceptacle d'un échantillon, et qui porte un insert destiné à être inséré sous la peau de l'animal, cet insert portant un cône de pénétration comportant des lames élastiques ici représentées déployées dans une configuration libre de la flèche, qui sont plaquées le long du dard creux tranchant lors de l'entrée de la flèche dans le corps de l'animal dans une position contrainte, et qui tendent ensuite à s'écarter radialement, empêchant la sortie de l'insert une fois inséré;
- la figure 2 illustre, de façon schématisée et en élévation, la flèche de la figure 1 ;
- la figure 3 illustre, de façon schématisée et en coupe axiale selon le plan de coupe AA de la figure 2, une première variante de la flèche de la figure 1, dans laquelle le dard creux tranchant est fixé au corps de flèche par une liaison vissée;
- la figure 4 illustre, de façon schématisée et en coupe axiale, un détail d'une deuxième variante de la flèche de la figure 1, dans laquelle le dard creux tranchant est fixé au corps de flèche par au moins un élément d'assemblage radial.

### Description détaillée de l'invention

Afin de permettre un balisage et un recueil d'échantillon sans dommage majeur pour l'animal, l'invention concerne un dispositif scientifique d'étude d'animaux 100, qui est agencé pour être lancé par un dispositif de propulsion sur un animal pour un balisage et un recueil d'échantillon de cet animal, comportant une flèche 1.

Cette flèche 1 comporte une partie rigide formant support, dite corps de flèche 2, et une pointe amovible. Le support est la partie structurelle utilisée pour la projection de la flèche 1 et pour son retrait après prélèvement d'un échantillon. La pointe amovible, dite dard creux tranchant 3, remplit deux fonctions: la pénétration dans le corps de l'animal, et le recueil d'échantillon.

De façon préférée, et telle que visible sur les figures, la pointe amovible porte l'insert amovible; dans une autre variante non illustrée, c'est le corps de flèche qui porte l'insert amovible.

Par «insert» on entend, au sens large, tout insert destiné à rester dans le corps de l'animal, par exemple tel qu'illustré sur les figures un élément d'accrochage 52 pour l'accrochage d'un câble 53 tractant une balise 60, notamment une balise de positionnement radio-émettrice, ou bien directement une telle balise insérée à demeure dans le corps de l'animal, ou/et un capteur, mais aussi par exemple un élément permettant de soigner et/ou refermer la plaie de l'animal; ainsi deux inserts, ou davantage, disposés l'un derrière l'autre sur la pointe amovible, peuvent être insérés dans l'animal en une seule opération.

Selon l'invention, cette flèche 1 comporte ainsi, sensiblement coaxiaux autour d'une direction longitudinale D, et démontables l'un par rapport à l'autre, un corps de flèche 2, un dard creux tranchant 3 et au moins un insert 5.

Le corps de flèche 2 comporte, dans une partie arrière 22 des moyens d'appui pour un dispositif de propulsion. Il porte en appui longitudinal dans une partie avant 21 le dard creux tranchant 3.

Ce dard creux tranchant 3 forme un tube de prélèvement agencé pour pénétrer la peau d'un animal lors d'un impact et carotter dans un alésage 4 du dard creux tranchant 3 un échantillon des tissus de cet animal.

Plus particulièrement, le dard creux tranchant 3 porte extérieurement au moins un insert 5, qui est agencé pour être fixée sous la peau de l'animal. Au moins un de ces inserts 5, ou l'insert 5 s'il est unique tel qu'illustré sur les figures, est amovible par rapport au corps de flèche 2 et au dard creux tranchant 3, et est agencé pour reposer en appui sur un premier épaulement frontal 6 du corps de flèche 2.

Le corps de flèche 2 comporte au niveau de la partie arrière 22 un deuxième épaulement 7, qui est agencé pour stopper la pénétration du corps de flèche 2 dans l'animal.

Plus particulièrement, l'insert 5 porte un cône de pénétration 50 extérieur au dard creux tranchant 3, comportant des lames élastiques 51 qui sont agencées pour se déployer radialement sous la peau de l'animal et empêcher la sortie de l'insert 5 après son introduction sous la peau de l'animal. Ces lames élastiques, à l'état libre, forment un cône en éventail, tel que visible en figure 1. Ces lames élastiques 51, qui sont déployées dans une configuration libre de la flèche, sont plaquées le long du dard creux tranchant lors de l'entrée de la flèche dans le corps de l'animal dans une position contrainte, et tendent ensuite à s'écarter radialement, empêchant la sortie de l'insert une fois inséré.

Plus particulièrement, le cône de pénétration 50 est en matière plastique. Avantageusement ce cône de pénétration 50 est arrêté longitudinalement par l'élément d'accrochage 52, ou un ergot radial, ou similaire, que comporte l'insert 5.

Dans une variante particulière, l'insert 5 est en matériau biodégradable, ou en matière plastique biodégradable, pour libérer la balise 60 après une durée déterminée.

Dans une autre variante, le corps de la balise 60 est en matériau biodégradable, ou en matière plastique biodégradable, pour libérer la balise 60 après une durée déterminée.

Dans une autre variante, le câble 53 tractant la balise 60 est en matériau biodégradable, ou en matière plastique biodégradable, pour libérer la balise 60 après une durée déterminée.

Plus particulièrement, chaque insert 5 est monté avec un ajustement glissant juste sur le dard creux tranchant 3.

Plus particulièrement, le corps de flèche 2 comporte des moyens de fixation avant 23 pour la fixation réversible du dard creux tranchant 3 lequel comporte des moyens de fixation avant complémentaires 32, et comporte, dans le prolongement de l'alésage 4, un canal d'évacuation 8 agencé pour le rejet de liquide par au moins une ouverture d'évacuation 9, à proximité immédiate des moyens de fixation avant 23. Chaque ouverture d'évacuation 9 s'étend obliquement depuis le canal d'évacuation 8 vers la périphérie du corps de flèche 2, depuis l'extrémité distale du dard creux tranchant 3 vers la partie arrière 22 du corps de flèche 2, et est ouverte dans le sens d'évacuation pour la faciliter. La flèche 1 est ainsi bien adaptée au cas des animaux marins, et comporte un chemin d'évacuation de l'eau très court ce qui diminue la surpression dans le tube lors de la rentrée du dard creux tranchant 3 dans la peau de l'animal, et augmente donc la probabilité et la quantité du prélèvement de tissus. La variante illustrée sur les figures comporte trois telles ouvertures d'évacuation 9 pour l'évacuation de l'eau, qui sont ouvertes dans le sens d'évacuation afin de la faciliter.

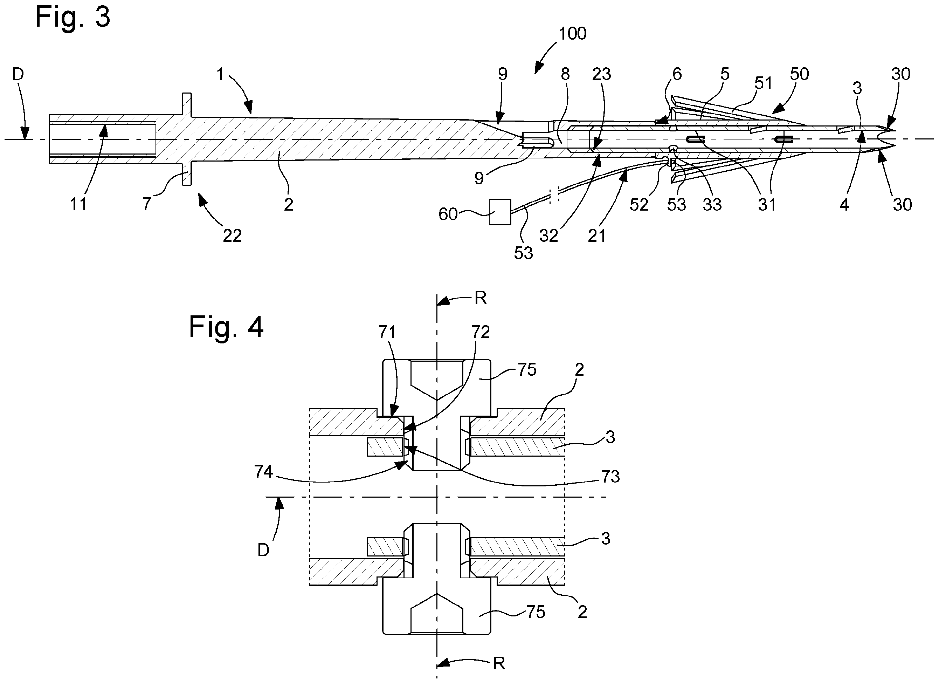

Plus particulièrement, dans la première variante de la figure 3, les moyens de fixation avant 23 comportent un taraudage agencé pour coopérer de façon complémentaire avec un des moyens de fixation avant complémentaires 32, notamment un filetage que comporte le dard creux tranchant 3, lequel comporte au moins une encoche de manoeuvre 33, par exemple un trou radial, pour faciliter son vissage ou son dévissage.

Plus particulièrement, dans la deuxième variante de la figure 4, le dard creux tranchant 3 est fixé au corps de flèche 2 par au moins un élément d'assemblage radial, tel qu'une vis radiale 75, une goupille, ou similaire. Par exemple les moyens de fixation avant 23 du corps de flèche 2 comportent au moins une implantation comportant un perçage radial 72, selon une direction radiale R, et un lamage d'appui 71, et les moyens de fixation avant complémentaires 32 comportent au moins un taraudage radial 73 selon une direction radiale R; la solidarisation entre le dard creux tranchant 3 et le corps de flèche 2 est alors assurée par au moins une vis radiale 75 comportant un filetage 74, agencée pour venir en appui sur un lamage d'appui 71, et venir en coopération avec un taraudage 73, au travers d'un perçage radial 72. La figure 4 représente deux telles vis radiales 75 antagonistes. Cette deuxième variante permet d'éviter d'affaiblir le dard creux tranchant par un filetage axial.

Plus particulièrement, la flèche 1 comporte, dans la partie arrière 22 et à l'opposé du dard creux tranchant 3, des moyens de fixation arrière 11 pour la fixation d'une perche ou d'un câble pour la récupération de la flèche 1.

Plus particulièrement, le dard creux tranchant 3 comporte une pluralité d'ardillons internes 31, ou/et l'alésage 4 du dard creux tranchant 3 comporte un profil intérieur rugueux, pour le maintien de l'échantillon de l'animal lors d'un retrait de la flèche 1. Plus particulièrement encore, tel que visible sur la figure 3, le dard creux tranchant 3 comporte une pluralité d'ardillons internes 31 disposés en hélicoïde, afin de minimiser l'affaiblissement mécanique. La présence d'ardillons 31 rentrant dans le tube sur la pointe permet de retenir les tissus lors de la rétractation de la flèche 1.

Plus particulièrement, le dard creux tranchant 3 comporte des arêtes de coupe 30 à sa partie distale avant, agencées pour perforer la peau de l'animal. Plus particulièrement encore, le dard creux tranchant 3 comporte au moins trois arêtes de coupe 30 à sa partie avant pour assurer une pénétration oblique sous la peau de l'animal. Ce design à trois arêtes de coupe en bout de pointe permet d'assurer qu'une arête de coupe touche en premier la peau quelle que soit l'orientation de la flèche 1 lors de son arrivée oblique sur la peau de l'animal.

Plus particulièrement, le dard creux tranchant 3 est interchangeable et est réalisé dans un matériau convenant à la pénétration dans la peau du type d'animal concerné. Il constitue une pointe amovible interchangeable permettant un choix de matière différent pour la pointe afin d'en maximiser les propriétés mécaniques et d'ainsi diminuer les risques de déformation lors de la pénétration dans la peau. Le taraudage 23 dans le corps de flèche 2 n'est de préférence pas affleurant à l'extrémité, afin que la zone la plus sollicitée mécaniquement du corps creux tranchant 3 ne soit pas filetée, on évite ainsi son affaiblissement.

Plus particulièrement, les matériaux utilisés pour la fabrication du corps de flèche 2 et du dard creux tranchant 3 des matériaux résistants à la corrosion en milieu marin, tels que par exemple de l'acier inoxydable, du titane, ou similaire. Un exemple de couple de matériaux intéressant est un corps de flèche 2 en acier inoxydable 304 (résistance à la corrosion avec usinabilité correcte) avec un dard creux tranchant en titane grade 5 (très bonne résistance à la corrosion avec haute limite élastique).

Plus particulièrement, le dispositif scientifique d'étude d'animaux 100 comporte des moyens de propulsion 200 agencés pour projeter la flèche sur une zone d'observation ou/et de balisage d'un tel animal, ou/et comporte des moyens de retrait 300 comportant une perche ou un câble pour la récupération de la flèche 1 enfermant l'échantillon de l'animal dans le dard creux tranchant 3, après l'insertion de l'insert 5 sous la peau de l'animal.

En somme, le dispositif selon l'invention permet de réaliser la pose d'une balise et le prélèvement d'un échantillon en une seule opération, de façon sécurisée, et en préservant au maximum l'animal étudié.

## Revendications

1. Dispositif scientifique d'étude d'animaux (100), agencé pour être lancé par un dispositif de propulsion sur un animal pour un balisage et un recueil d'échantillon dudit animal, comportant une flèche (1), **caractérisé en ce que** ladite flèche (1) comporte, sensiblement coaxiaux autour d'une direction longitudinale (D) et démontables l'un par rapport à l'autre, un corps de flèche (2), un dard creux tranchant (3) et au moins un insert (5), ledit corps de flèche (2) comportant dans une partie arrière (22) des moyens d'appui pour un dit dispositif de propulsion et portant en appui longitudinal dans une partie avant (21) ledit dard creux tranchant (3) formant un tube de prélèvement agencé pour pénétrer la peau d'un animal lors d'un impact et carotter dans un alésage (4) dudit dard creux tranchant (3) un échantillon dudit animal, ledit dard creux tranchant (3) portant extérieurement ledit au moins un insert (5) agencé pour être fixé sous la peau dudit animal, ledit au moins un insert (5) étant amovible par rapport audit corps de flèche (2) et audit dard creux tranchant (3) et agencé pour reposer en appui sur un premier épaulement frontal (6) dudit corps de flèche (2), lequel corps de flèche (2) comporte au niveau de ladite partie arrière (22) un deuxième épaulement (7) agencé pour stopper la pénétration dudit corps de flèche (2) dans ledit animal.

2. Dispositif scientifique d'étude d'animaux (100) selon la revendication 1, **caractérisé en ce que** ledit insert (5) porte un cône de pénétration (50) extérieur audit dard creux tranchant (3), comportant des lames élastiques (51) agencées pour se déployer radialement sous la peau dudit animal et empêcher la sortie du dit insert (5) après son introduction sous la peau dudit animal.

3. Dispositif scientifique d'étude d'animaux (100) selon la revendication 2, **caractérisé en ce que** ledit cône de pénétration (50) est en matière plastique.

4. Dispositif scientifique d'étude d'animaux (100) selon l'une des revendications 1 à 3, **caractérisé en ce que** ledit insert (5) comporte un élément d'accrochage (52) pour l'accrochage d'un câble (53) tractant une balise (60).

5. Dispositif scientifique d'étude d'animaux (100) selon la revendication 4, **caractérisé en ce que** ledit insert (5) et/ou ledit câble (53) et/ou le corps de ladite balise (60) est en matériau biodégradable, ou en matière plastique biodégradable, pour libérer ladite balise (60) après une durée déterminée.

6. Dispositif scientifique d'étude d'animaux (100) selon l'une des revendications 1 à 5, **caractérisé en ce que** chaque dit insert (5) est monté avec un ajustement glissant juste sur ledit dard creux tranchant (3).

7. Dispositif scientifique d'étude d'animaux (100) selon l'une des revendications 1 à 6, **caractérisé en ce que** ledit corps de flèche (2) comporte des moyens de fixation avant (23) pour la fixation réversible dudit dard creux tranchant (3) lequel comporte des moyens de fixation avant complémentaires (32), et comporte, dans le prolongement dudit alésage (4), un canal d'évacuation (8) agencé pour le rejet de liquide par au moins une ouverture d'évacuation (9) à proximité immédiate desdits moyens de fixation avant (23), chaque dite ouverture d'évacuation (9) s'étendant obliquement depuis ledit canal d'évacuation (8) vers la périphérie dudit corps de flèche (2), depuis l'extrémité distale dudit dard creux tranchant (3) vers ladite partie arrière (22) dudit corps de flèche (2) et étant ouverte dans le sens d'évacuation pour la faciliter.

8. Dispositif scientifique d'étude d'animaux (100) selon la revendication 7, **caractérisé en ce que** lesdits moyens de fixation avant (23) comportent un taraudage agencé pour coopérer de façon complémentaire avec un filetage (32) que comporte ledit dard creux tranchant (3), lequel comporte au moins une encoche de manoeuvre (33) pour faciliter son vissage ou son dévissage.

9. Dispositif scientifique d'étude d'animaux (100) selon la revendication 7, **caractérisé en ce que** ledit dard creux tranchant (3) est fixé audit corps de flèche (2) par au moins un élément d'assemblage radial (75).

10. Dispositif scientifique d'étude d'animaux (100) selon la revendication 9, **caractérisé en ce que** lesdits moyens de fixation avant (23) dudit corps de flèche (2) comportent au moins une implantation comportant un perçage radial (72), selon une direction radiale (R), et un lamage d'appui (71), et lesdits moyens de fixation avant complémentaires (32) comportent au moins un taraudage radial (73) selon une direction radiale (R), la solidarisation entre ledit dard creux tranchant (3) et ledit corps de flèche (2) étant assurée par au moins une vis radiale (75) comportant un filetage (74), agencée pour venir en appui sur un dit lamage d'appui (7, et venir en coopération avec un dit taraudage radial (73), au travers d'un dit perçage radial (72).

11. Dispositif scientifique d'étude d'animaux (100) selon l'une des revendications 1 à 10, **caractérisé en ce que** ladite flèche (1) comporte, dans ladite partie arrière (22) et à l'opposé dudit dard creux tranchant (3), des moyens de fixation arrière (11) pour la fixation d'une perche ou d'un câble pour la récupération de ladite flèche (1).

12. Dispositif scientifique d'étude d'animaux (100) selon l'une des revendications 1 à 11, **caractérisé en ce que** ledit dard creux tranchant (3) comporte une pluralité d'ardillons internes (31), ou/et **en ce que** ledit alésage (4) dudit dard creux tranchant (3) comporte un profil intérieur rugueux, pour le maintien dudit échantillon dudit animal lors d'un retrait de ladite flèche (1).

13. Dispositif scientifique d'étude d'animaux (100) selon la revendication 12, **caractérisé en ce que** ledit dard creux tranchant (3) comporte une pluralité d'ardillons internes (31) disposés en hélicoïde dans ledit alésage (4).

14. Dispositif scientifique d'étude d'animaux (100) selon l'une des revendications 1 à 13, **caractérisé en ce que** ledit dard creux tranchant (3) comporte des arêtes de coupe (30) à sa partie distale avant agencées pour perforer la peau dudit animal.

15. Dispositif scientifique d'étude d'animaux (100) selon la revendication 14, **caractérisé en ce que** ledit dard creux tranchant (3) comporte au moins trois dites arêtes de coupe (30) à sa dite partie avant pour assurer une pénétration oblique sous la peau dudit animal.

16. Dispositif scientifique d'étude d'animaux (100) selon l'une des revendications 1 à 15, **caractérisé en ce que** ledit dard creux tranchant (3) est interchangeable et est réalisé dans un matériau convenant à la pénétration dans la peau du type d'animal concerné.

17. Dispositif scientifique d'étude d'animaux (100) selon l'une des revendications 1 à 16, **caractérisé en ce que** ledit corps de flèche (2) est en acier inoxydable 304 et **en ce que** ledit dard creux tranchant (3) est en titane grade 5.

18. Dispositif scientifique d'étude d'animaux (100) selon l'une des revendications 1 à 16, **caractérisé en ce que** ledit dispositif scientifique d'étude d'animaux (100) comporte des moyens de propulsion (200) agencés pour projeter ladite flèche sur une zone d'observation ou/et de balisage d'un dit animal, ou/et comporte des moyens de retrait (300) comportant une perche ou un câble pour la récupération de ladite flèche (1) enfermant ledit échantillon dudit animal dans ledit dard creux tranchant (3), après l'insertion dudit insert (5) sous la peau dudit animal.
